# EUROPÄISCHE PATENTSCHRIFT

(11) **EP 2 215 967 B1**
(45) Veröffentlichungstag und Bekanntmachung des Hinweises auf die Patenterteilung: **30.11.2011**
(21) Anmeldenummer: 09169625.2
(22) Anmeldetag: 07.09.2009
(51) Int. Cl.: A61B 6/10, A61N 5/10, G21F 3/00

(54) **Dentalprotektor**
Dental protector
Protecteur dentaire

(43) Veröffentlichungstag der Anmeldung: 11.08.2010
(73) Patentinhaber: Hibrand Establishment, 9494 Schaan (LI)
(72) Erfinder: Bettega, Remo, 9444, Diepoldsau (CH)
(74) Vertreter: Winter, Brandl, Fürniss, Hübner, Röss, Kaiser, Polte - Partnerschaft

(56) Entgegenhaltungen:
- WO-A-98/01865
- US-A- 4 223 229
- US-A1- 2003 178 028

## Beschreibung

Die vorliegende Erfindung betrifft einen Dentalprotektor zur Verhinderung einer Schädigung von gesundem Gewebe in unmittelbarer Umgebung eines Tumors durch Bestrahlung im Rahmen einer Tumortherapie.

Zur Behandlung bestimmter Tumoren wie z. B. Plattenepithelkarzinomen oder Adenokarzinomen der Kopf-Hals-Region, insbesondere der Region des Mundbodens oder auch der Nasennebenhöhlen, ist die Bestrahlung nicht nur von Teilen, sondern von der gesamten Zunge, also auch ihrer Randbereiche, indiziert. In Ruhelage, d. h. in Okklusionsstellung, überragt die Zunge normalerweise die Unterkieferzähne und liegt am harten Gaumen an. Bei einer Bestrahlung ohne Änderung der Zungenposition würden daher die Zähne, insbesondere die Zahnkronen mitbestrahlt, was zu schweren Schäden des Zahnschmelzes und in weiterer Folge sogar zum Verlust von zuvor gesunden Zähne führen kann. Darüber hinaus ist eine so genannte Osteoradionekrose der Kieferknochen - also ein strahlungseffiziertes und irreparables Absterben (Nekrose) von Knochensubstanz - insbesondere nach Extraktion eines Zahnes aus dem bestrahlten Knochen beobachtet worden, was mitunter zu einem Verlust großer Kieferanteile und somit, neben den damit unmittelbar verbundenen medizinischen und Alltagsproblemen, zu einer deutlichen Einbuße der Lebensqualität des Patienten führt, also zu einer medizinisch-induzierten physischen ebenso wie einer psychischen Beeinträchtigung. Die Dauer einer Strahlenbehandlung hängt z. B. von der Art und Lage des Tumors ab. Es können bis zu 30 Sitzungen erforderlich sein, so dass sich die Behandlung über einige Wochen erstrecken kann, wobei die reine Bestrahlungszeit in einer Sitzung ca. 2 bis 3 Minuten beträgt.

Es ist somit eine Aufgabe der vorliegenden Erfindung, eine Vorrichtung - hierin als Dentalprotektor bezeichnet - vorzuschlagen, wobei die Vorrichtung zweierlei leistet:
1. Sie soll dazu geeignet sein, die Zunge während einer Sitzung nach unten (kaudal) drücken und so niederhalten, so dass die von ihr sonst überlagerten Zahnkronen nicht mehr im Strahlenfeld liegen und folglich durch die Bestrahlung kein gesundes Gewebe geschädigt wird.
2. Die durch die Vorrichtung festgelegte Lage der Zunge im Mundraum soll reproduzierbar sein, so dass die Zunge aufgrund der durch die Vorrichtung erreichten immer gleichen Positionierung in jeder von mehreren Sitzungen wohl definiert und präzise bestrahlt werden kann.

Diese Aufgabe wird durch die Merkmale des Anspruchs 1 gelöst.

Gemäß einem Aspekt der vorliegenden Erfindung umfasst ein Dentalprotektor zum Schutz der Ober- und Unterkieferzähne eines Patienten bei externer Strahlentherapie (Teletherapie) einen Kieferteil zur Erzeugung einer Bisshebung, d. h. einer vertikalen Erhöhung des Abstandes zwischen Ober- und Unterkiefer, und einen mit dem Kieferteil verbundenen Zungenteil zum Niederhalten der Zunge des Patienten, wobei das Kieferteil eine Öffnung zur Sichtkontrolle der Zungenposition des Patienten umfasst. Die Lage des Tumors bestimmt die optische Achse der Bestrahlung, die durch die Öffnung führen kann, aber nicht muss, da das Kieferteil zumindest im Frontbereich vorzugsweise aus einem Material gebildet ist, das kein Bestrahlungshindernis darstellt, die verwendete(n) Wellenlänge(n) also nur unwesentlich absorbiert und streut. Die erfindungsgemäß verwendete Strahlung ist vorzugsweise elektromagnetische Mehrfeldstrahlung mit einer Energie in der Größenordnung von 6 MeV, die tief (bis zu 2 cm) in das Gewebe eindringen kann. Die Angabe von 6 MeV stellt jedoch nur eine große Orientierung dar, der verwendete Wert kann im Einzelfall von diesem Wert abweichen. Der Kieferteil des erfindungsgemäßen Dentalprotektors erfüllt zwei Aufgaben. Er soll zum einen in Verbindung mit dem Zungenteil eine Bisshebung erzeugen und so die Oberkieferzähne aus dem Strahlenfeld rücken. Zum anderen dient er als Befestigungsmittel, um eine exakte Position des Dentalprotektors reproduzierbar und lagestabil herzustellen. Vorteilhafterweise wird die Zunge durch den Zungenteil unter die Zahnebene gedrückt.

Gemäß einer vorteilhaften Ausgestaltung der vorliegenden Erfindung ist das Kieferteil allgemein zahnbogenförmig ausgebildet, so dass der Druck zur Aufrechterhaltung der Bisshebung vorteilhafterweise auf alle Oberkieferzähne, im Falle eines Unbezahnten auf den gesamten Zahnfleischbogen, gleichmäßig verteilt ist. Darüber kann hierdurch auf einfache Weise eine exakte Position im Mund des Patienten definiert und eine Reproduzierbarkeit dieser Position gewährleistet werden, so dass der Kieferteil während der Sitzung in dieser Position bleibt und bei folgenden Sitzungen wieder in diese Position gebracht werden kann.

Es ist zu beachten, dass durch die hierin gewählte Terminologie, die auf (zahn-) medizinische Lage- und Richtungsbezeichnungen (z. B. "kaudal") sowie die allgemeine Anatomie des Menschen (z. B. "zahnbogenförmig") Bezug nimmt, implizit eine korrekte Position am Menschen in eingesetztem Zustand des Dentalprotektors bzw. eine an die Anatomie des Menschen angepasste Formgebung definiert ist.

Gemäß einer vorteilhaften Ausgestaltung der vorliegenden Erfindung ist die Öffnung in dem Kieferteil median angeordnet. Alternativ kann die Öffnung aus dieser Position nach distal versetzt sein, um so z. B. die Sicht auf einen eher am seitlichen Zungenrand gelegenen Tumor zu verbessern.

Gemäß einer vorteilhaften Ausgestaltung der vorliegenden Erfindung ist ein kaudaler Rand des Kieferteils durch einen sich von der Öffnung kaudal erstreckenden Spalt zweigeteilt. Durch diese Zweiteilung wird das Sichtfeld des Strahlentherapeuten nach unten erweitert, ohne auf die oben genannten Vorteile verzichten zu müssen. Dies ist insbesondere aufgrund der Position der Zunge vorteilhaft, die während der Behandlung durch den Zungenteil niedergedrückt ist, sich also an einer tieferen Position befindet. Die Breite des Spalts ist ein Kompromiss zwischen Stabilität und Steifigkeit des erfindungsgemäßen Dentalprotektors einerseits und einem möglichst großen Sichtfeld andererseits, und entspricht gemäß einer vorteilhaften Ausgestaltung der vorliegenden Erfindung der lateralen Erstreckung der unteren Schneidezähne des Patienten; d. h. durch den Kieferteil wird die Unterkieferzahnreihe nur von den Zähnen 38-33 und 48-43 (bezüglich der Zähne wird stets die übliche zahnmedizinische Nummerierung verwendet) eingefasst. Erfindungsgemäß ist der kraniale Rand des Kieferteils stets durchgehend, weist also keinen sich entsprechend von der Öffnung nach kranial erstreckenden Spalt auf. Es werden somit die durch den erfindungsgemäßen Dentalprotektor alle Zähne 38-33 und 48-43 des Oberkiefers eingefasst, was zur Stabilität und Handhabung des Dentalprotektors beiträgt.

Gemäß einer vorteilhaften Ausgestaltung der vorliegenden Erfindung sind der Kieferteil und der Zungenteil lösbar miteinander verbunden. Dies hat zum einen den Vorteil, dass beide Teile unabhängig voneinander hergestellt werden können, die Herstellung somit auf unterschiedliche Materialien für den Kiefern- und den Zungenteil angepasst werden kann, und zum anderen den Vorteil, dass dadurch das Einsetzen des erfindungsgemäßen Dentalprotektors in den Mund des Patienten erleichtert ist, indem beide Teile nacheinander in den Mund eingeführt und in situ miteinander verbunden werden.

Die Verbindung zwischen dem Kieferteil und dem Zungenteil ist gemäß einer vorteilhaften Ausgestaltung der vorliegenden Erfindung nach Art eines Matrizen-Patrizen-Systems ausgebildet, wobei beide Teile intraoral durch Ineinanderstecken der einen geeigneten Vorsprung aufweisenden Patrize mit der eine komplementär ausgebildete Vertiefung aufweisenden Matrize eine Presspassung bildend miteinander verbunden werden.

Gemäß einer vorteilhaften Ausgestaltung der vorliegenden Erfindung ist die Matrize im Zungenteil und die Patrize im Kieferteil ausgebildet. Die umgekehrte Variante ist jedoch äquivalent. Die Matrizen-Patrizen-Verbindung erstreckt sich entlang des Kontaktbereichs zwischen dem Kieferteil und dem Zungenteil, d. h. im Falle einer zahnbogenförmigen Ausbildung des Kiefer- und Zungenteils ebenfalls zahnbogenförmig, vorteilhafterweise im Wesentlichen auf deren gesamter Länge von z. B. ca. 40 mm nach Art einer Nut-Feder-Verbindung, wobei in einer zu der Längserstreckung senkrechten Schnittebenenansicht der Kontaktbereich zwischen dem Kiefer- und dem Zungenteil vorzugsweise als S-förmig gekrümmte Linie erscheint. Die Matrizen-Patrizen-Verbindung kann jedoch auf jede beliebige andere Art ausgebildet sein, solange - wie es in der oben beschriebenen Ausgestaltung der vorliegenden Erfindung der Fall ist - ein Verrutschen, d. h. eine Relativbewegung zwischen dem Kieferteil und dem Zungenteil während der Behandlung ausgeschlossen ist.

Gemäß einer vorteilhaften Ausgestaltung der vorliegenden Erfindung ist mit der Patrize des Zungenteils ein Zungenniederhalteabschnitt, der die eigentliche Funktion des Zungenhalteteils ausübt, verbunden, wobei diese Verbindung vorteilhafterweise einteilig ist.

Der Zungenniederhalteabschnitt ist gemäß einer vorteilhaften Ausgestaltung der vorliegenden Erfindung im Frontalschnitt im Wesentlichen zungenförmig oder löffelartig, so dass die Zunge durch ihn anatomisch gleichmäßig, insbesondere flächig und nicht punktuell niedergedrückt wird. Der Zungenniederhalteabschnitt kann die Zunge vollständig oder nur teilweise, z. B. nur einen vorderen oder nur einen hinteren Teil, überdecken. Wie es oben schon erwähnt ist, ist der Zungenniederhalteabschnitt vorzugsweise aus einem Material gebildet, das für den Wellenlängenbereich der verwendeten Strahlung im Wesentlichen transparent und nicht streuend ist, wobei die Eigenschaft der Transparenz die Energiemenge bestimmt, die insgesamt durch den erfindungsgemäßen Dentalprotektor hindurchtritt, und die Eigenschaft möglichst geringer Streuung eine hohe Energiedichte in einem wohl definierten und möglichst kleinen Bestrahlungsbereich ergibt.

Gemäß einer vorteilhaften Ausgestaltung der vorliegenden Erfindung ist der Kieferteil und / oder der Zungenteil aus einem festen Kunststoff gebildet, wobei "fest" hierin bedeutet, dass eine konstante Bisshebung und konstante räumliche Relationen zwischen dem Kieferteil, dem Zungenteil - und somit zwischen dem Ober- und dem Unterkiefer - und der Zunge gewährleistet ist. Dies schließt jedoch eine gewisse Formelastizität nicht aus. Im Gegenteil. Um die oben beschriebenen Funktionen optimal erfüllen zu können, ist es vorteilhaft, dass die Zähne die Oberfläche des Kiefer- bzw. Zungenteils geringfügig elastisch eindrücken.

Gemäß einer vorteilhaften Ausgestaltung der vorliegenden Efindung ist der Dentalprotektor individuell an den Patienten angepasst. Dadurch werden sowohl die Präzision des chirurgischen Eingriffs und die Reproduzierbarkeit der relativen Positionen von Oberkiefer, Unterkiefer und Zunge in den einzelnen Sitzungen, als auch der Tragekomfort des Dentalprotektors für den Patienten, was zu einer Reduzierung z. B. von Schleimhautirritationen beiträgt, erhöht.

Gemäß einer vorteilhaften Ausgestaltung der vorliegenden Erfindung wird der Schutz von gesundem Gewebe dadurch verbessert, dass das Kieferteil so ausgebildet ist, dass durch das Kieferteil die Zahnkronen von wenigstens einem Teil der Ober- und Unterkieferzähne wenigstens teilweise umschlossen ist. Welche Zahnkronen dies betrifft, hängt u. a. von der Lage des Tumors und von der Zahl der Zähne ab.

Die obigen und weitere Aufgaben, Eigenschaften und Vorteile der vorliegenden Erfindung sind aus der nachfolgenden detaillierten Beschreibung, die unter Bezugnahme auf die beigefügten Zeichnungen gemacht wurde, deutlicher ersichtlich. Die Erfindung ist durch Ansprüche definiert. Die Ausführungsbeispiele, die nicht im Rahmen der Ansprüche liegen, sind nur als Veranschaulichung angegeben und sind nicht Teil der Erfindung. In den Zeichnungen sind:
Fig. 1 eine Vorderansicht eines Dentalprotektor gemäß einer Ausführungsform der vorliegenden Erfindung; und
Fign. 2 bis 4 der Dentalprotektor nach Fig. 1, eingesetzt in einen menschlichen Schädel, in einer Vorderansicht, einer Seitenansicht und einer Rückansicht.

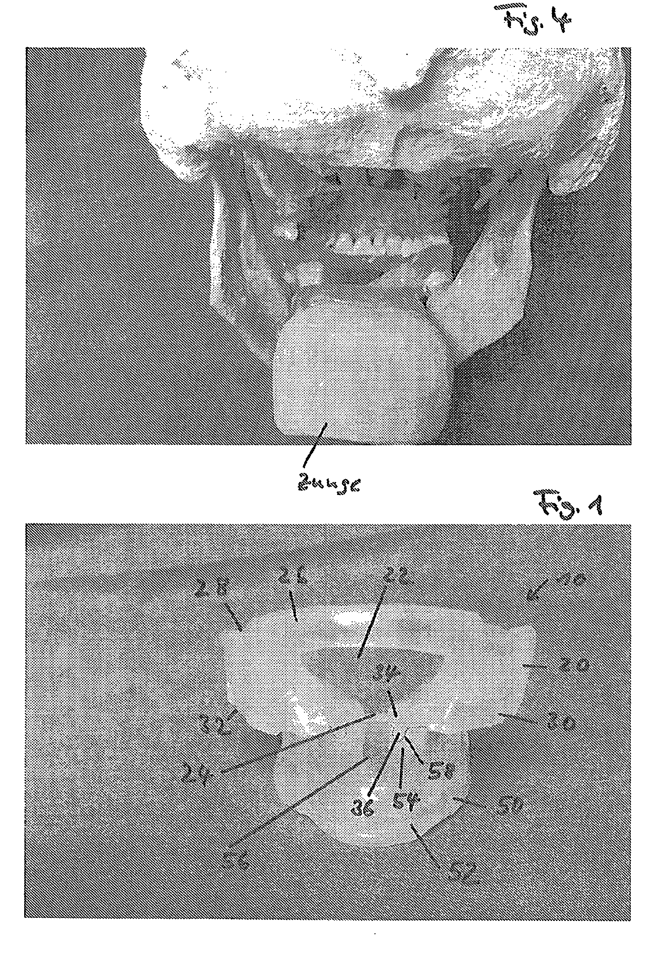

Fig. 1 zeigt eine Vorderansicht eines Dentalprotektors gemäß einer Ausführungsform der vorliegenden Erfindung, der allgemein mit 10 bezeichnet ist. Der Dentalprotektor 10 umfasst einen Kieferteil 20 und einen Zungenteil 50.

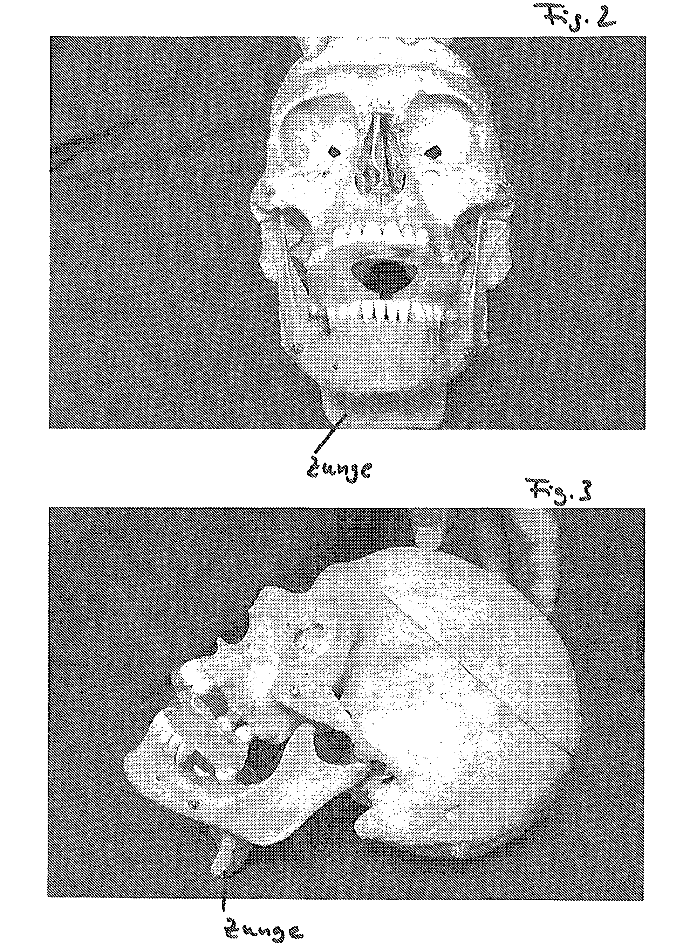

Der Kieferteil 20 ist in seiner Längserstreckung zahnbogenförmig ausgebildet, d. h. er erstreckt sich in eingesetztem Zustand entlang des oberen und unteren Zahnbogens, wie es in den Fign. 2 bis 4 gezeigt ist. Der Kieferteil 20 weist ferner in seinem frontalmedialen Abschnitt eine allgemein dreieckförmige Öffnung 22 auf, die kaudal (in Fig. 1 nach unten) in einen nach unten offenen Spalt 24 übergeht. Der Kieferteil 20 weist einen Oberkieferabschnitt 26 mit einer oberen Zahnauflage- oder Stützfläche 28 und einen mit dem Oberkieferabschnitt 26 einteilig verbundenen Unterkieferabschnitt 30 mit einer unteren Zahnauflage- oder Stützfläche 32 auf. Der Unterkieferabschnitt 30 weist ferner einen sich nach lingual erstreckenden Patrizenabschnitt 34 auf, von dem ein sich ebenfalls entlang der gesamten Länge des Patrizenabschnitts 34 erstreckender Steg 36 kaudal hervorragt. Durch die Öffnung 22 und den Spalt 24 ist der Unterkieferabschnitt 30 einschließlich dessen Patrizenabschnitt 34 in einen linken und einen rechten Abschnitt geteilt. Der Unterkieferabschnitt 30 fasst daher bei der Anwendung an einem vollbezahnten Patienten nur die Unterkieferzähne 38-33 und 48-43 ein; die unteren Schneidezähne sind ausgespart. Der Oberkieferabschnitt 26 hingegen ist durchgehend ausgebildet, so dass er bei der Anwendung an dem vollbezahnten Patienten sämtliche Zähne 18-11 und 28-21 einfasst.

Der Zungenteil 50 umfasst einen Zungenniederhalteabschnitt 52 und einen einteilig mit diesem verbundenen Matrizenabschnitt 54. Der Matrizenabschnitt 54 weist einen vertikalen Spalt 56 auf, durch den er in einen linken und einen rechten Abschnitt geteilt ist. Der Matrizenabschnitt 54 weist ferner eine zu dem Steg 36 des Patrizenabschnitts 34 komplementär ausgebildete Nut 58 auf, in der in einem in den Mund des Patienten eingesetzten Zustand des erfindungsgemäßen Dentalprotektors 10 der Steg 36 so aufgenommen ist, dass eine lösbare, für die Behandlung jedoch ausreichend stabile Verbindung nach Art einer Matrizen-Patrizen- oder Nut-Feder-Verbindung zwischen dem Kieferteil 20 und dem Zungenteil 50 gebildet ist. Der Zungenniederhalteabschnitt 52 ist an die Wölbung des Zungenrückens angepasst und dazu geeignet, die Zunge an einer wohl definierten und reproduzierbaren Position unterhalb der Unterkieferzähne niederzuhalten. Wie es in Fig. 1 gezeigt ist, setzt sich der Spalt 24 des Oberkieferabschnitts 30 des Kieferteils 20 durch den Matrizenabschnitt 54 in den Zungenniederhalteabschnitt 52 fort. Wie es in Fig. 1 gezeigt ist, ist in einem Querschnitt durch die Matrizen-Patrizen-Verbindung die Kontaktfläche zwischen dem Matrizenabschnitt 54 und dem Patrizenabschnitt 34 als S-förmige Linie sichtbar, die das Ineinandergreifen des Stegs 36 und der Nut 58 zeigt.

Die Fign. 2 bis 4 zeigen den Dentalprotektor 10 der Fig. 1 in eingesetztem Zustand. Deutlich erkennbar ist die durch den Dentalprotektor 10 erreichte Bisshebung und Niederdrückung der Zunge, die eine zahnunbedenkliche Bestrahlung unter Sichtkontrolle durch die Offnunge 22 und den Schlitz 24 ermöglicht.

Gemäß der Ausführungsform ist der Dentalprotektor 10 aus einem festen, aber von den Zähnen oberflächig leicht elastisch eindrückbaren Material gebildet, das nahezu verlustfrei durchstrahlt werden kann. Im Übrigen kann der Dentalprotektor 10 nach jeder Sitzung einfach, z. B. mit Hilfe einer Zahnbürste und Wasser, gereinigt werden.

Der Dentalprotektor 10 gemäß der Ausführungsform kann zum Beispiel mit einem Verfahren hergestellt werden, das allgemein die folgenden Schritte umfasst:
- Herstellen von Abdrücken des Oberkiefers, des Unterkiefers und der Zunge des Patienten;
- Herstellen von Gipsmodellen des Oberkiefers, des Unterkiefers und Zunge des Patienten anhand der Abdrücke;
- Gelenkiges Verbinden des Oberkiefer- und Unterkiefermodells und Einstellen einer wohl definierten Bisshebung zur Herstellung eines Bisshebungsmodells;
- Herstellen des Kieferteils anhand des Bisshebungsmodells; und
- Herstellen des Zungenteils.

Hierbei kann die Herstellung des Oberkieferabdrucks und des Zungenabdrucks gleichzeitig erfolgen. Ferner kann zur Herstellung des Oberkieferabdrucks und des Zungenabdrucks ein Oberkieferabformlöffel verwendet werden, auf dessen Oberseite der Oberkiefer und auf dessen Unterseite die Zunge abgeformt wird. Das Kieferteil kann vorteilhafterweise mittels Kunststoffstreutechnik hergestellt werden. Vorteilhafterweise kann in dem Schritt der Herstellung des Kieferteils der Schritt der Einpolymerisierung der Patrize enthalten sein. Zur Herstellung des Zungenteils ist es ferner vorteilhaft, dass das Gipsmodell der Zunge so in das Gipsmodell des Unterkiefers eingelegt wird, dass das Gipsmodell der Zunge unterhalb der Zahnkronen des Gipsmodells des Unterkiefers angeordnet ist, und der Zungenteil auf dem Zungenrücken des Gipsmodells der Zunge aufpolymerisiert wird.

Konkret kann das Verfahren zur Herstellung des erfindungsgemäßen Dentalprotektors zum Beispiel wie folgt durchgeführt werden:
Zuerst werden Alginatabdrücke vom Ober- und Unterkiefer beim Patienten genommen. Hierzu wird das Abdruckmaterial mit exakt 20°C warmem Wasser angerührt. Anschließend wird der Abdrucklöffel, der aus Weichplastik besteht, mit dem Material in den Mund des Patienten eingebracht und dort für 10 Minuten belassen. Nach Entnahme muss mindestens eine Stunde die Rückstellung abgewartet werden bevor die Abdrücke ausgegossen werden können.

Für das Ausgießen der Modelle wird Alabastergips verwendet, der im Mischungsverhältnis 200g Gips / 75ml Wasser, 30 Grad warm, angerührt wird.

Die Bissnahme erfolgt beim Zahnlosen unter Zuhilfenahme eventuell vorhandener Prothesen, sonst mit eigens aus lichtgehärtetem Kunststoff angefertigten Bissschablonen mittels Pfeilregistrat nach Gerber. Beim Bezahnten werden zwei 1,5 cm lange Wachsstreifen im Molarenbereich als Aufbissregistrat verwendet.

Nach Erstellen des Registrats in zentrischer Position muss dieses durch nochmalige Bissnahme überprüft werden. Insgesamt müssen fünf Versuche in zentrischer Position reproduzierbar sein. Danach wird die Position des Oberkiefers mittels Gesichtsbogen in einen Artikulator, einem Gerät zur Simulation von Kiefergelenksbewegungen, transferiert.

Im Anschluss daran werden die Gipsmodelle im Artikulator montiert, hierzu wird mit 3%iger Bikarbonatlösung versetztes Wasser zum Befestigen der Gipsmodelle genommen.

Zur Herstellung des Gerätes wird smaragdblauer, durchsichtiger Kunststoff mit eingearbeiteten Lufteinschlüssen verwendet. Die Komgrösse beträgt 100-110 Mikrometer. Nach dem Anrühren muss eine Anteigzeit von genau 60 Minuten vergehen, bevor der Kunststoff weiterverarbeitet werden kann. Im nächsten Schritt werden die Lufteinschlüsse mittels Druckluft eingebracht. Danach wird der Kunststoff unmittelbar ohne Isoliermittel auf das Gipsmodell im Artikulator aufgebracht und mittels lanzettförmigem Nickel-Titanspatel modelliert, wobei darauf zu achten ist, dass auf dem Oberkiefermodell eine genau 5 mm breite Furche entlang des Kieferkammes entsteht, die nicht tiefer als 5 mm sein darf. Zuvor wurde der Incisalstift um 45 mm erhöht.

Die anschließende Ausarbeitung erfolgt aufgrund des o.a. Kunststoffes mittels 5µ kreuzverzahnten Hartmetallfräsen in absteigendem Durchmesser des Arbeitsteils bei einer Drehzahl von 80000U/min.

Eine anschließende Politur erfolgt mittels kanarischem Vulkanbimsstein für mindestens 20 Minuten ohne Wasserkühlung, um eine bessere Endpolymerisation zu erreichen. Danach erfolgt noch eine 31 Minuten dauernde Nachpolitur mittels 80%iger Schlämmkreide.

Zum Abschluss des Herstellungsprozesses erfolgt eine Kontrolle. Hierzu wird unter dem Elektronenmikroskop unter 100000-facher Vergrößerung die Ausrichtung der Kunststoffkugeln überprüft, um eine Strahlenstreuung beurteilen zu können.

Schließlich wird das Gerät am Patienten angepasst, d. h. es werden die Bisshöhe, die zentrische Relation und eventuell vorhandene Druckstellen erfaßt und entsprechende Korrekturen durchgeführt.

Obgleich die vorliegende Erfindung bezüglich der bevorzugten Ausführungsformen offenbart worden ist, um ein besseres Verständnis von diesen zu ermöglichen, sollte wahrgenommen werden, dass die Erfindung auf verschiedene Weisen verwirklicht werden kann, ohne den Umfang der Erfindung zu verlassen. Deshalb sollte die Erfindung derart verstanden werden, dass sie alle möglichen Ausführungsformen und Ausgestaltungen zu den gezeigten Ausführungsformen beinhaltet, die realisiert werden können, ohne den Umfang der Erfindung zu verlassen, wie er in den beigefügten Ansprüchen dargelegt ist.

### Bezugszeichenliste

- 10: Dentalprotektor
- 20: Kieferteil
- 22: Öffnung in 20
- 24: Spalt in 20
- 26: Oberkieferabschnitt
- 28: Obere Zahnauflage- oder Stützfläche
- 30: Unterkieferabschnitt
- 32: Untere Zahnauflage- oder Stützfläche
- 34: Patrizenabschnitt
- 36: Steg an 34
- 50: Zungenteil
- 52: Zungenniederhalteabschnitt
- 54: Matrizenabschnitt
- 56: Spalt
- 58: Nut in 54

## Patentansprüche

1. Dentalprotektor (10) zum Schutz der Ober- und Unterkieferzähne eines Patienten bei externer Strahlentherapie, mit einem Kieferteil (20) zur Erzeugung einer Bisshebung, **dadurch gekennzeichnet, dass** der dentalprotektor einen
mit dem Kieferteil (20) verbundenen Zungenteil (50) zum Niederhalten der Zunge des Patientern umfasst, wobei das Kieferteil (20) eine Öffnung (22) zur Sichtkontrolle der Zungenposition des Patienten umfasst.

2. Dentalprotektor (10) nach Anspruch 1, wobei der Kieferteil (20) allgemein zahnbogenförmig'ausgebildet ist.

3. Dentalprotektor (10) nach Anspruch 1 oder 2, wobei die Öffnung (22) median angeordnet ist.

4. Dentalprotektor (10) nach einem der Ansprüche 1 bis 3, wobei ein kaudaler Rand des Kieferteils (20) durch einen sich von der Öffnung (22) erstreckenden Spalt (24) zweigeteilt ist.

5. Dentalprotektor (10) nach Anspruch 4 wobei die Breite des Spalts (24) der lateralen Erstreckung der unteren Schneidezähne des Patienten entspricht.

6. Dentalprotektor (10) nach einem der Ansprüche 1 bis 5 wobei die Verbindung zwischen dem Kieferteil (20) und dem Zungenteil (50) lösbar ist.

7. Dentalprotektor (10) nach einem der Ansprüche 1 bis 5, wobei die lösbare Verbindung nach Art eines Matrizen-Patrizen-Systems ausgebildet ist.

8. Dentalprotektor (10) nach Anspruch 7, wobei die Matrize im Zungenteil (54) ausgebildet ist und eine Längsrichtung aufweist, die zahnbogenförmig gekrümmt ist und entlang der eine in einem zu der Längsrichtung senkrechten Querschnitt S-förmige Nut (58) ausgebildet ist, und dass die Patrize (34) im Kieferteil (20) ausgebildet ist und einen zu der S-bogenförmigen Nut (58) komplementär ausgebildeten Steg (26) aufweist.

9. Dentalprotektor (10) nach Anspruch 7 oder 8, wobei der Zungenteil (50) einen mit der Patrize (34) verbundenen Zungenniederhalteabschnitt (52) umfasst.

10. Dentalprotektor (10) nach Anspruch 9, wobei der Zungenniedernalteabschnitt (52) einen im Wesentlichen zungenförmigen Frontalschnitt aufweist.

11. Dentalprotektor (20) nach einem der Ansprüche 1 bis 10, wobei der Kieferteil (20) und / oder der Zungenteil (50) aus einem festen Kunststoff gebildet
ist.

12. Dentalprotektor (10) nach einem der Ansprüche 1 bis 11, wobei der Dentalprotektor (10) individuell an den Patienten angepasst ist.

13. Dentalprotektor (10) nach einem der Ansprüche 1 bis 12, wobei das Kieferteil (20) so ausgebildet ist, dass durch das Kierferteil (20) die Zahnkronen von wenigstens einem Teil der Ober- und Unterkieferzähne wenigstens teilweise umschließbar sind.

## Claims

1. A dental protector (10) for protecting a patient's upper and lower jaw teeth during teletherapy, comprising a jaw part (20) for generating a raised bite, **characterized in that** the dental protector comprises a tongue part (50) for depressing the patient's tongue which is connected to the jaw part (20), said jaw part (20) having an opening (22) for visual inspection of the patient's tongue position.

2. The dental protector (10) according to claim 1, wherein the jaw part (20) is realized generally in the shape of a dental arch.

3. The dental protector (10) according to claim 1 or 2, wherein the opening (22) is arranged in a median position.

4. The dental protector (10) according to any one of claims 1 to 3, wherein a caudal rim of the jaw part (20) is bisected by a slit (24) extending from the opening (22).

5. The dental protector (10) according to claim 4, wherein the width of the slit (24) corresponds to the lateral extension of the patient's lower incisors.

6. The dental protector (10) according to any one of claims 1 to 5, wherein the connection between the jaw part (20) and the tongue part (50) is detachable.

7. The dental protector (10) according to any one of claims 1 to 5, wherein the detachable connection is realized in the manner of a male part - female part system.

8. The dental protector (10) according to claim 7, wherein the female part is formed in the tongue part (54) and has a longitudinal direction curved in a dental arch shape and along which an groove (58) is formed that is S-shaped in a cross-section orthogonal to said longitudinal direction, and the male part (34) is formed in the jaw part (20) and has a longitudinal protrusion (36) formed complementarily with respect to the S-shaped groove (58).

9. The dental protector (10) according to claim 7 or 8, wherein the tongue part (50) comprises a tongue depressing portion (52) which is connected to the male part (34).

10. The dental protector (10) according to claim 9, wherein the tongue depressing portion (52) has a substantially tongue-shaped frontal cross section.

11. The dental protector (10) according to any one of claims 1 to 10, wherein the jaw part (20) and/or the tongue part (50) is formed of a firm synthetic material.

12. The dental protector (10) according to any one of claims 1 to 11, wherein the dental protector (10) is individually adapted to the patient.

13. The dental protector (10) according to any one of claims 1 to 12, wherein the jaw part (20) is configured in such a way that the crowns of at least some of the teeth of the upper and lower jaws may be enclosed at least partly by the jaw part (20).

## Revendications

1. Protecteur dentaire (10) pour protéger les dents de la mâchoire supérieure et de la mâchoire inférieure d'un patient lors d'une radiothérapie externe, comprenant un élément pour mâchoire (20) destiné à produire une surélévation occlusale, **caractérisé en ce que** le protecteur dentaire comprend un élément pour langue (50) relié à l'élément pour mâchoire (20) et destiné à maintenir baissée la langue du patient, sachant que l'élément pour mâchoire (20) comprend une ouverture (22) pour le contrôle visuel de la position de la langue du patient.

2. Protecteur dentaire (10) selon la revendication 1, sachant que l'élément pour mâchoire (20) est réalisé en forme générale d'arcade dentaire.

3. Protecteur dentaire (10) selon la revendication 1 ou 2, sachant que l'ouverture (22) est disposée en position médiane.

4. Protecteur dentaire (10) selon l'une quelconque des revendications 1 à 3, sachant qu'un bord caudal de l'élément pour mâchoire (20) est divisé en deux par une fente (24) s'étendant à partir de l'ouverture (22).

5. Protecteur dentaire (10) selon la revendication 4, sachant que la largeur de la fente (24) correspond à l'étendue latérale des incisives inférieures du patient.

6. Protecteur dentaire (10) selon l'une quelconque des revendications 1 à 5, sachant que la liaison entre l'élément pour mâchoire (20) et l'élément pour langue (50) est amovible.

7. Protecteur dentaire (10) selon l'une quelconque des revendications 1 à 5, sachant que la liaison amovible est réalisée à la manière d'un système matrice-patrice.

8. Protecteur dentaire (10) selon la revendication 7, sachant que la matrice est réalisée dans l'élément pour langue (54) et présente une direction longitudinale qui est incurvée en forme d'arcade dentaire et le long de laquelle est réalisée une rainure (58) en forme de S dans une coupe transversale à la direction longitudinale, et sachant que la patrice (34) est réalisée dans l'élément pour mâchoire (20) et présente une nervure (36) réalisée de manière complémentaire à la rainure (58) en forme d'arc en S.

9. Protecteur dentaire (10) selon la revendication 7 ou 8, sachant que l'élément pour langue (50) comprend une partie (52) pour maintenir la langue baissée, laquelle partie est reliée à la patrice (34).

10. Protecteur dentaire (10) selon la revendication 9, sachant que la partie (52) pour maintenir la langue baissée présente une coupe frontale essentiellement en forme de langue.

11. Protecteur dentaire (10) selon l'une quelconque des revendications 1 à 10, sachant que l'élément pour mâchoire (20) et/ou l'élément pour langue (50) sont constitués d'une matière plastique rigide.

12. Protecteur dentaire (10) selon l'une quelconque des revendications 1 à 11, sachant que le protecteur dentaire (10) est individuellement adapté au patient.

13. Protecteur dentaire (10) selon l'une quelconque des revendications 1 à 12, sachant que l'élément pour mâchoire (20) est réalisé de telle sorte que l'élément pour mâchoire (20) peut enserrer au moins partiellement les couronnes dentaires d'au moins une partie des dents de la mâchoire supérieure et de la mâchoire inférieure.
